# EUROPÄISCHE PATENTANMELDUNG

(11) **EP 1 862 197 A2**
(43) Veröffentlichungstag der Anmeldung: **05.12.2007**
(21) Anmeldenummer: 07009570.8
(22) Anmeldetag: 12.05.2007
(51) Int. Cl.: A61P 3/00, A61P 3/10, A23J 3/16, A23J 3/08, A23L 1/305

(54) **Verwendung eines Proteinkonzentrates**

(30) Priorität: 31.05.2006 DE 102006025338
(71) Anmelder: TROUILLÈ, André, 29553 Bienenbüttel (DE)
(72) Erfinder: TROUILLÈ, André, 29553 Bienenbüttel (DE)
(74) Vertreter: Kloiber, Thomas

(57) **Zusammenfassung**

Es wird die Verwendung eines proteinreichen Nahrungsmittelkonzentrates als diätetisches Lebensmittel vorgeschlagen, um Patienten mit Stoffwechselstörungen oder Erkrankungen, die mit Stoffwechselstörungen assoziiert sind, zu behandeln. Studien zeigen, dass die Konzentrat-Gabe zu einer hochsignifikanten Normalisierung zahlreicher Parameter führt, die als Risikofaktoren oder Marker für Herz-Kreislauferkrankungen und Atherosklerose gelten.

## Beschreibung

Die Erfindung betrifft die Verwendung eines leicht verdaulichen Proteinkonzentrats zur Behandlung von Stoffwechselstörungen.

Bei Patienten mit einer Grunderkrankung wie zum Beispiel Diabetes mellitus liegen häufig auch Störungen des Stoffwechsels vor. So ist die diabetische Erkrankung u.a. durch Störungen des Lipid-, Kohlenhydrat-und Insulinstoffwechsels charakterisiert. Um diese Stoffwechselprozesse im Hinblick auf den Krankheitsverlauf günstig zu beeinflussen, ist zur Unterstützung die Einhaltung einer Diät notwendig. Die Diät kann bei leichteren Erkrankungen alleine ausreichend sein oder eine medikamentöse Behandlung unterstützen. Im Rahmen der Diät erfolgt dabei ein weitgehender Verzicht auf tierische Produkte, wie Fisch, Fleisch, Wurst, Eiern, da diese Nahrungsmittel hohe Anteile an Cholesterin und Triglyceriden aufweisen. Nachteilig ist an der Diät, dass die dadurch verursachte geringere Aufnahme von Proteinen zu einem Mangel an essentiellen Aminosäuren führen kann. Diese Aminosäuren können vom Körper nicht selbst synthetisiert werden. Gerade bei Stoffwechselerkrankungen müssen die essentiellen Aminosäuren in aber ausreichender Menge sowie im richtigen Verhältnis aufgenommen werden.

Es ist daher die Aufgabe der vorliegenden Erfindung, ein Nahrungsmittel vorzuschlagen, welches die Nachteile einer herkömmlichen Diät vermeidet und als diätetisches Nahrungsmittel in der Lage ist, die metabolische Lage von Patienten mit Stoffwechselstörungen zu verbessern.

Diese Aufgabe wird erfindungsgemäß dadurch gelöst, dass ein proteinreiches Nahrungsmittelkonzentrat verwendet wird, dessen Herstellung in der EP 1 049 384 B1 beschrieben ist. Das Konzentrat hat einen auf das Gesamtgewicht bezogenen Eiweißanteil von mindestens 50%, wobei das Eiweiß vorzugsweise zu mindestens 40% als freie Aminosäuren oder kurzkettige Peptide vorliegt.

Durch den hohen Anteil von freien Aminosäuren und kurzkettigen Peptiden ist das Nahrungsmittelkonzentrat besonders leicht verdaulich, weil die Proteine bereits als Bestandteile vorliegen, die im Darm gut resorbiert werden. Dieses erhöht die Verträglichkeit, was besonders für erkrankte Personen wichtig ist.

Das Proteinkonzentrat wird aus einem pflanzlichen Ausgangsprodukt hergestellt, vorzugsweise Soja oder einem Sojaprodukt, insbesondere mit einem Proteinanteil von 60 bis 95 %, oder einem tierischen Ausgangsprodukt, vorzugsweise Milch oder einem Milchprodukt, oder einem Gemisch aus den genannten pflanzlichen und tierischen Ausgangsprodukten, wobei das Ausgangsprodukt oder das Gemisch mit einem Emulgator, vorzugsweise Lecithin in einer auf die Gesamtmenge bezogenen Menge von 1 bis 6 %, vorzugsweise 2 bis 4 %, imprägniert und dann in das das so imprägnierte proteinreiche Proteinkonzentrat Honig mit einem auf die Gesamtmenge bezogenen Anteil von 10 bis 40 %, vorzugsweise 25 bis 30 %, eingerührt ist, welcher vorzugsweise flüssig ist oder als wässrige Lösung vorliegt und in sehr feinem Strahl, vorzugsweise aus mehreren Düsen und insbesondere drucklos, zugegeben wird, wobei eine Temperatur von 60° C, vorzugsweise Raumtemperatur, nicht überschritten wird, und das Proteinkonzentrat nach dem Einrühren mehrere Stunden stehen gelassen wird, vorzugsweise bis zum Erreichen eines Feuchtigkeitsgehalts von 1 bis 8 %, insbesondere 2 bis 5%.

Es wird auf diese Weise ein gelbliches Pulver von leicht nussigem Geschmack erhalten, das einen Feuchtigkeitsgehalt von ca. 7% aufweist. Die Ausbeute ist praktisch quantitativ. Die während des Vermischens beobachtete Erwärmung führt nicht zu einer Zerstörung insbesondere der essentiellen Aminosäuren. Das Pulver weist folgenden Aminosäuregehalt auf:

| Aminosäure | g/100g |
|---|---|
| Alanin | 1,9 |
| Arginin | 3,8 |
| Asparaginsäure | 6,1 |
| Cystein | 3,5 |
| Glutaminsäure | 10,6 |
| Glycin | 2,9 |
| Histidin | 1,1 |
| Isoleucin | 1,2 |
| Leucin | 4,7 |
| Lysin | 3,1 |
| Methionin | 2,1 |
| Phenylalanin | 3,1 |
| Prolin | 2,6 |
| Serin | 5,7 |
| Threonin | 2,3 |
| Tryptophan | 0,30 |
| Tyrosin | 2,7 |
| Valin | 2,4 |

Es hat sich gezeigt, dass das nach dem oben beschriebenen Verfahren hergestellte Proteinkonzentrat geeignet ist, Patienten mit Stoffwechselstörungen oder mit Erkrankungen, welche mit Stoffwechselstörungen assoziiert sind, zu behandeln. Dies können zum Beispiel Störungen des Aminosäurestoffwechsels sein. Durch den hohen Anteil an freien Aminosäuren wirkt das Konzentrat Mangelerscheinungen entgegen, indem ausreichend essentielle und nichtessentielle Aminosäuren zur Verfügung gestellt werden. Der Einfluss des Konzentrates ist aber nicht auf diese Wirkung beschränkt, da die verschiedenen physiologischen Stoffwechselprozesse regulativ und über Substrataustausch gekoppelt sind. So können Aminosäuren Vorstufen für andere Stoffwechselprodukte seien. Asparaginsäure kann beispielsweise die Synthese von ATP stimulieren, indem es in Oxalacetat umgewandelt und dann in den Citratzyklus eingeschleust wird. Neben diesem direkten Effekt der Inhaltsstoffe beeinflusst das Proteinkonzentrat durch seine diätetische Wirkung auch andere gestörte physiologische Prozesse, wie den Lipid- und Kohlenhydratstoffwechsel.

Mit dem Nahrungsmittelkonzentrat lässt sich Diabetes mellitus, vorzugsweise vom Typ 2, zu behandeln. Die Wirkung beruht darauf, dass die Stoffwechsellage von Diabetikern verbessert und insbesondere der Glukosespiegel gesenkt wird.

### Dieses wurde durch die folgende Studie gezeigt:

Jeweils 30 nicht insulinpflichtige unter oraler Medikation und/oder Diätplan stehende Frauen, mittleres Alter 63,3 Jahre, und Männer, mittleres Alter 63,8 Jahre, haben täglich 50 Gramm des Konzentrates ohne weitere Veränderung der Medikation oder des Lebensstils zu sich genommen. Zu Beginn der Studie wurden bei den Teilnehmern laborübliche Blutparameter wie Nüchternglukose, Insulin, HbA1C und das Körpergewicht gemessen. Die Glukosetoleranz wurde nach 12-stündigem Fasten mit einem Trunk aus 75 Gramm Glukose in 150 ml Wasser nach einer und zwei Stunden bestimmt. Die Messung von Nüchternglukose und Insulin wurde nach 8 und 20 Wochen wiederholt und die Messung der Parameter Nüchternglukose, HbA1C, Insulin, Gewicht und Glukosetoleranz wurde nach jeweils 14 und 26 Wochen wiederholt. Innerhalb von 26 Wochen sanken der Nüchternblutglukosespiegel von durchschnittlich 167 mg/dl auf 127 mg/dl, der HbA1C von 8,2 auf 7,3 Prozent und der nüchtern gemessene Insulinspiegel von 20,7 mU/ml auf 10,6 mU/ml. Das Gewicht der Diabetiker reduzierte sich während der Studiendauer von 26 Wochen um 2,5 kg bis 3,7 kg.

Weiterhin kann das Proteinkonzentrat für die Behandlung von Patienten mit Hyperlipidämie verwendet werden. Dieses wurde durch die folgende Studie gezeigt:
Bei 12 übergewichtigen Personen (7 Männer, 5 Frauen, Alter 34-66 J., BMI >25 kg/m²) wurden im Rahmen einer therapeutisch indizierten Gewichtsintervention zu Beginn und nach 4 Wochen folgende Kenngrößen bestimmt:
   Anthropometrische Daten: Körpergröße, Körpergewicht, Körperfett, BMI. Lipidstatus: Cholesterin, TG, HDL, LDL, VLDL, Apo Al, Apo All, Apo B, Lp (a).
   Begleitende proinflammatorische Faktoren: Leptin, Insulin, CRP, Fibrinogen.
   Begleitende metabolische Regulation: TSH, STH.

Eine renale oder hepatische Funktionsstörung oder hormonelle Ursache des Übergewichts wurde ausgeschlossen. Während einer zunächst 4-wöchigen Interventionsphase erhielten die Teilnehmer eine individuelle, auf ihr Normalgewicht abgestimmte Dosierung des erfindungsgemäßen Proteinkonzentrates. Neben der morgendlichen und abendlichen Einnahme des Diätnahrungsmittels wurde mittags die Zufuhr einer fettreduzierten Normalmahlzeit gestattet. Die Tageskalorienzufuhr lag damit bei ca. 1.200 kcal/d.

Die Parameter des Lipidstoffwechsels, die am Anfang und nach vierwöchiger Gabe des Proteinkonzentrates gemessen wurden, zeigt Tabelle 1; signifikante Unterschiede sind entsprechend p < 0,05 bzw. < 0,01 mit dem Symbol * bzw. ** gekennzeichnet:

| Tab. 1 | Beginn | 4 Wochen |
|---|---|---|
| | | |
| Gesamt-Chol. (mg/dl) | 199.0 ± 28.7 | 161.0 ± 26.7 ** |
| Triglyzeride (mg/dl) | 160.0 ± 53.4 | 125.0 ± 63.4* |
| HDL-Chol. (mg/dl) | 48.4 ± 11.4 | 45.0 ± 7.5 |
| LDL-Chol. (mg/dl) | 112.7 ± 25.4 | 87.6 ± 22.9* |
| VLDL-Chol. (mg/dl) | 33.7 ± 10.6 | 23.5 ± 7.42* |
| Apo Al (mg/dl) | 160.0 ± 20.2 | 148.0 ± 24.1 |
| Apo All (mg/dl) | 47.0 ± 5.3 | 38.4 ± 5.9** |
| Apo B (mg/dl) | 109.4 ± 25.9 | 84.6 ± 18.3** |
| Lp-a-Chol. (mg/dl) | 4.0 ± 7.3 | 5.3 ± 9.5 |

Hormone der Gewichtsregulation und begleitende proinflammatorische und metabolisch-hormonale Parameter sind in Tabelle 2 aufgeführt (Symbole wie in Tab. 1):

| Tab. 2 | Beginn | 4 Wochen |
|---|---|---|
| | | |
| Leptin (ng/ml) | 24.60± 15.80 | 13.80 ± 13.20** |
| Insulin (mU/ml) | 14.20± 9.30 | 7.30 ± 9.00* |
| CRP (mg/dl) | 0.24± 0.20 | 0.13 ± 0.12* |
| Fibrinogen (mg/dl) | 371.00± 49.40 | 366.00 ± 61.40 |
| TSH (µU/ml) | 0.90± 0.87 | 0.72 ± 0.74 |
| STH (ng/ml) | 0.89± 1.48 | 0.65 ± 0.74 |

Während der Intervention und der damit verbundenen Gewichtsreduktion sinkt der Leptinspiegel deutlich ab. Dabei wird unter der mit dem Konzentrat-Gabe der Leptinspiegel stärker gesenkt als unter der mit herkömmlicher Diät erzielten Gewichtsabnahme. Da erhöhte Leptinspiegel als Ursache für Adipositas und metabolische Störungen, z.B. Diabetes, angesehen werden, kann damit ein zentraler Risikofaktor positiv beeinflusst werden.

Wie die Tabelle 1 außerdem zeigt, wird unter dem Einfluss des Proteinkonzentrates die Insulinkonzentration im Blut signifikant gesenkt. Erhöhte Insulinwerte gelten als atherogener und inflammatorischer Risikofaktor.

Weiterhin tritt eine hoch signifikante Absenkung im Gesamt- und LDL-Cholesterin ein. Zusätzlich zum atherogenen Lipidprofil sind auch Veränderungen im proinflammatorischen Profil zu beobachten. Das für die Blutgefäßwände besonders atherogene Lipoprotein Apo-B wird in den Gruppen, die Konzentrat erhielten, signifikant deutlicher gesenkt als in der Vergleichsgruppe.

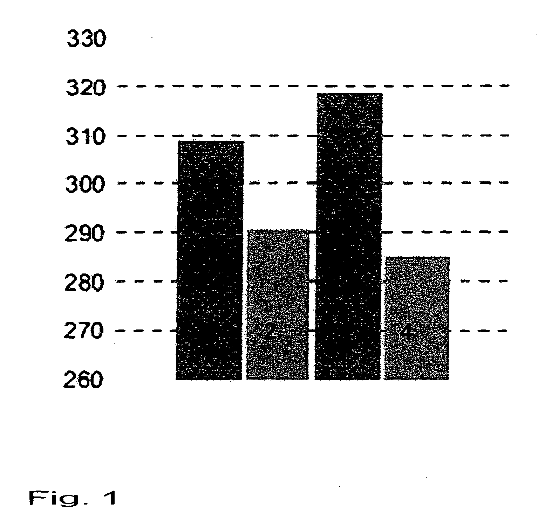

Die Figur 1 zeigt eine Balkengrafik, die den Body Mass Index (BMI) in kg/m² und den Körperfettanteil in Prozent jeweils vor und nach der Intervention, also der Einnahme des Proteinkonzentrates, wiedergibt. Balken 1 zeigt den BMI und Balken 3 den Körperfettanteil vor der Intervention, Balken 2 den BMI und Balken 4 den Körperfettanteil nach der Intervention. Die Figur offenbart, dass bei allen untersuchten Personen nach 4 Wochen eine signifikante Gewichtsabnahme zu verzeichnen war. Diese lag im Mittel bei 4,8 kg.

Diese Ergebnisse lassen sich so zusammenfassen, dass das proteinreiche Nahrungsmittelkonzentrat überraschend günstige Wirkungen auf die Stoffwechsellage von Patienten mit Diabetes mellitus oder Hyperlipidämie hat.

Die Konzentrat-Gabe führt zu einer hochsignifikanten Normalisierung zahlreicher Parameter, die als Risikofaktoren oder Marker für Herz-Kreislauferkrankungen und Atherosklerose gelten.

## Patentansprüche

1. Verwendung eines proteinreiches Nahrungsmittelkonzentrates mit einem auf das Gesamtgewicht bezogenen Eiweißanteil von mindestens 50%, welcher vorzugsweise zu mindestens 40% als freie Aminosäuren oder kurzkettige Peptide vorliegt, hergestellt aus einem pflanzlichen Ausgangsprodukt, vorzugsweise Soja oder einem Sojaprodukt, insbesondere mit einem Proteinanteil von 60 bis 95 %, oder einem tierischen Ausgangsprodukt, vorzugsweise Milch oder einem Milchprodukt, oder einem Gemisch aus den genannten pflanzlichen und tierischen Ausgangsprodukten, wobei das Ausgangsprodukt oder das Gemisch mit einem Emulgator, vorzugsweise Lecithin in einer auf die Gesamtmenge bezogenen Menge von 1 bis 6 %, vorzugsweise 2 bis 4 %, imprägniert und dann in das das so imprägnierte proteinreiche Proteinkonzentrat Honig mit einem auf die Gesamtmenge bezogenen Anteil von 10 bis 40 %, vorzugsweise 25 bis 30 %, eingerührt ist, welcher vorzugsweise flüssig ist oder als wässrige Lösung vorliegt und in sehr feinem Strahl, vorzugsweise aus mehreren Düsen und insbesondere drucklos, zugegeben wird, wobei eine Temperatur von 60° C, vorzugsweise Raumtemperatur, nicht überschritten wird, und das Proteinkonzentrat nach dem Einrühren mehrere Stunden stehen gelassen wird, vorzugsweise bis zum Erreichen eines Feuchtigkeitsgehalts von 1 bis 8 %, insbesondere 2 bis 5%, als diätetisches Lebensmittel zur Behandlung von Stoffwechselstörungen oder Erkrankungen, die mit Stoffwechselstörungen assoziiert sind.

2. Verwendung eines proteinreichen Nahrungsmittelkonzentrates nach Anspruch 1, **dadurch gekennzeichnet, dass** die Erkrankung Diabetes mellitus, vorzugsweise vom Typ 2, ist.

3. Verwendung eines proteinreichen Nahrungsmittelkonzentrates nach Anspruch 1, **dadurch gekennzeichnet, dass** die Erkrankung eine Hyperlipidämie ist.
